# EUROPEAN PATENT SPECIFICATION

(11) **EP 3 383 449 B1**
(45) Date of publication and mention of the grant of the patent: **29.07.2020**
(21) Application number: 16871685.0
(22) Date of filing: 02.12.2016
(51) Int. Cl.: A61M 1/00, A61M 1/10, A61M 1/14, A61M 1/16, A61M 1/18, A61M 37/00

(54) **AN IMPLANTABLE RENAL REPLACEMENT THERAPY**
IMPLANTIERBARE NIERENERSATZTHERAPIE
THÉRAPIE DE REMPLACEMENT RÉNAL IMPLANTABLE

(30) Priority: 04.12.2015 US 201562262915 P
(43) Date of publication of application: 10.10.2018
(73) Proprietor: Qidni Labs, Inc., Buffalo, NY 14202 (US)
(72) Inventor: AHMADI, Morteza, San Francisco, CA 94103 (US)
(74) Representative: Kazi, Ilya
(86) International application number: PCT/US2016/064856
(87) International publication number: WO 2017/096325

(56) References cited:
- WO-A1-2008/086477
- WO-A2-2004/062710
- CN-A- 105 771 011
- US-A1- 2003 097 086
- US-A1- 2004 019 312
- US-A1- 2012 289 881
- US-A1- 2013 199 998

## Description

### FIELD

The present invention is in the technical field of medical devices and renal replacement therapies.

### BACKGROUND

There are nearly three million patients with kidney failure (end-stage renal disease) around the world. Patients' survival depend on dialysis, a thrice a week renal replacement therapy that they undergo until they receive a kidney transplant. The mortality rate of patients under dialysis is more than 60% in five years. On average, a year of dialysis of each patient costs upwards of $82,000 in the United States. Patients have to use dialysis for five to seven years on average before they find a kidney donated for transplantation. Thrice a week dialysis imposes lifestyle restrictions on patients and they endure a lot more hardships from complications due to dialysis treatment.

An implantable renal replacement therapy can provide access to treatment continuously and automatically 24/7 and removes the hardship of going to the dialysis clinics. In general, it can provide a more normal life for kidney patients and it can be a temporary solution for the patients in need of a kidney transplant until they find a kidney donated for transplantation.

Kidneys receive 25% of cardiac output. The total blood volume passes through kidneys every 4-5 minutes. Kidneys produce 180 liters of fluid per day and reabsorbs 178.5 liters of it, generating 1.5 liter of acidic (pH∼6) urine per day which also contains uremic toxins. WO 2008/086477 A1 discloses an implantable hemofiltration/reabsorption device comprising a pump between the hemofiltration chamber and the reabsorption chamber, wherein the filters are designed to prevent or reduce pore-fouling.

### SUMMARY OF THE DISCLOSURE

This invention describes a medical device which is an implantable renal replacement therapy for patients with end stage renal disease.

Blood is diverted from iliac artery (or another artery) into a filtration system which removes uremic toxins and excess water from blood. Blood is passed back into iliac vein (or another vein) and the uremic toxins and excess water go to the bladder.

The device has (i) a filtration system which blocks passage of blood proteins such as albumin but removes uremic toxins and excess water and (ii) a re-absorption system to re-claim some salt and water back into the blood from the filtrate of the filtration system. The device is connected to a control unit outside the body of the patient through a percutaneous driveline. The percutaneous driveline has (i) wires to electronics inside the body of the patient and (ii) at least one tube to transfer washing fluid to clean the filters and open the pores in case of clogging. Patient also carries batteries to power the device.

In general, in one embodiment, a renal replacement therapy system, including (1) a filtration system having a feed side, a filtrate side and a non-silicon ceramic filter separating the feed side and the filtrate side; (2) a re-absorption system having a feed side, a filtrate side and a non-silicon ceramic filter separating the feed side and the filtrate side; (3) a valveless blood flow circuit in communication with an arterial graft, an inlet to the filtration system feed side, an outlet to the filtration system feed side, an inlet to the re-absorption system filtrate side, an outlet to the re-absorption system filtrate side and a venous graft; (4) a percutaneous drive line including a fluid supply channel, a fluid return channel, a power cable, and a data communications cable; (5) a filtration system inlet valve in communication with the fluid supply channel and an inlet to the filtration system filtrate side; (6) a re-absorption system first outlet valve in communication with the re-absorption system feed side and the fluid return channel; (7) a re-absorption system second outlet valve in communication with the re-absorption system feed side and a graft in communication with a bladder or a urine collection bag ; (8) a filtrate pump with an inlet in communication with the filtration system filtrate side and an outlet in communication with the re-absorption system feed side; and (9) an implantable housing containing the filtration system, the re-absorption system, and the filtrate pump.

The implantable housing can be completely sealed except for four openings.

The four openings can be positioned to provide communication to an interior portion of the implantable housing for the percutaneous drive line, the blood flow circuit inlet to the filtration system feed side; the blood flow circuit outlet to the re-absorption system filtrate side and an opening in communication with the re-absorption system second outlet valve.

The implantable housing can be completely sealed except for five openings.

The five openings can be positioned to provide communication to an interior portion of the implantable housing for a fluid supply line, a fluid return line, the blood flow circuit inlet to the filtration system feed side; the blood flow circuit outlet to the re-absorption system filtrate side and an opening in communication with the re-absorption system second outlet valve.

The filtration system non-silicon ceramic filter can be selected to filter blood components less than 66,500 Daltons into the filtration system filtrate side.

The re-absorption system non-silicon ceramic filter can be sized to pass components within the filtrate feed side having a molecular weight of less than 500 Daltons into the re-absorption system filtrate side and into the valveless blood flow circuit.

The filtration system non-silicon ceramic filter can be selected to maintain substantially all albumin within the blood flowing within the valveless blood flow circuit flowing from the inlet to the filtration system feed side through the outlet to the filtration system feed side while passing through to the filtration system filtrate side molecular weight fluids having a molecular weight below 65,000 Dalton.

The filtration system non-silicon ceramic filter can contain one or more of aluminum oxide, zinc oxide, titanium oxide, and pyrolytic carbon.

The filtration system filter can contain one or more of the materials and structures with fracture toughness KIC values of higher than 1.00 (MPa . m1/2)

The re-absorption system non-silicon ceramic filter can contain one or more of aluminum oxide, zinc oxide, titanium oxide, and pyrolytic carbon.

The filtration system can include the fluid supply channel, the fluid return channel, the power cable, and the data communications cable of the percutaneous drive line are enclosed within a single biocompatible sheath.

The filtration system can include one or more of a sensor to measure a pressure within the filtration system; a sensor to measure a flow within the filtration system; a sensor to measure a pressure within the re-absorption system; or a sensor to measure a flow within the re-absorption system.

The filtration system can include one or more of a sensor to measure a quantity of a blood protein, a sensor to measure a quantity of a blood glucose, a sensor to measure a quantity of a blood urea or a sensor to measure a quantity of a blood creatinine within the filtration system.

The filtration system can include one or more of a sensor to measure a quantity of a blood protein, a sensor to measure a quantity of a blood glucose, a sensor to measure a quantity of a blood urea or a sensor to measure a quantity of a blood creatinine within the re-absorption system.

The system can include a sensor to indicate fouling or fracture of the non-silicon ceramic filter in the filtration system or a sensor to indicate fouling or fracture of the non-silicon ceramic filter in the re-absorption system.

In general, in one embodiment, the system can further include a control unit with an electronic display and a computer controller in electronic communication with the filtration system inlet valve, the re-absorption system first outlet valve, the re-absorption system second outlet valve and the filtrate pump.

The computer controller can be in electronic communication with one or more of the sensors.

The system can further include a blood pump with an inlet in communication with the arterial graft and an outlet in communication with the inlet to the filtration system feed side.

The system can include the computer controller which can further include a computer readable instruction for operating the system in a preselected functioning mode selected from a filtration mode, a re-absorption mode, a filter cleaning mode and a urine flushing mode.

The pre-selected functioning mode can be the filtration mode with the computer readable instruction for operating the system which can further include instructions to close the filtration system inlet valve, the re-absorption system first outlet valve, the re-absorption system second outlet valve while controlling the operation of the filtrate pump to pump filtrate from the filtration system to the re-absorption system.

The pre-selected functioning mode can be the re-absorption mode with the computer readable instruction for operating the system which can further include instructions to close the filtration system inlet valve, the re-absorption system first outlet valve, the re-absorption system second outlet valve while controlling the operation of the filtrate pump to pump filtrate through the re-absorption system non-silicon ceramic filter.

The pre-selected functioning mode can be the filter cleaning mode with the computer readable instruction for operating the system which can further include instructions to open the filtration system inlet valve, open the re-absorption system first outlet valve, close the re-absorption system second outlet valve while controlling the operation of the filtrate pump to pump a cleaning fluid.

The pre-selected functioning mode can be the urine flushing mode with the computer readable instruction for operating the system which can further include instructions to close the filtration system inlet valve, close the re-absorption system first outlet valve, open the re-absorption system second outlet valve while controlling the operation of the filtrate pump to pump filtrate from the re-absorption system to the and the graft in communication with the bladder or a urine collection bag.

The pre-selected functioning mode can be the service mode with the computer readable instruction for operating the system and van further include instructions to open or close the filtration system inlet valve, open or close the re-absorption system first outlet valve, open or close the re-absorption system second outlet valve while controlling the operation of the filtrate pump to pump or not to pump the filtrate from the re-absorption system to the and the graft in communication with the bladder.

The system can include the computer readable instruction for operating the system which can further include controlling operation of the blood pump during a pre-selected functioning mode.

The system can further include at least one battery pack connected to the control unit.

The controller display can further include a touch screen selecting one of the pre-selected functioning modes.

In general, in one embodiment, a method of performing a renal replacement filtration therapy includes (1) flowing blood from a patient arterial graft into a valveless blood flow circuit that flows through an inlet to a filtration system feed side, an outlet to a filtration system feed side, an inlet to a re-absorption system filtrate side, an outlet to a re-absorption system filtrate side and a venous graft; (2) filtering a portion of the components in the flowing blood in the filtration system feed side through a first non-silicon ceramic filter to provide a filtrate into a filtration system filtrate side; (3) pumping a portion of the filtrate from the filtration system filtrate side to a feed side of a re-absorption system; and (4) filtering a portion of the filtrate in the feed side of the re-absorption system through a second non-silicon ceramic filter to provide a portion of the filtrate components into the re-absorption system filtrate side in communication with the blood flow circuit.

The method can further include (1) performing one or more of the flowing, filtering, pumping and filtering steps by operating a filtration system inlet valve in communication with a fluid supply channel of a percutaneous drive line and an inlet to the filtration system filtrate side, (2) a re-absorption system first outlet valve in communication with the re-absorption system feed side and a fluid return channel of the percutaneous drive line, (3) a re-absorption system second outlet valve in communication with the re-absorption system feed side and a graft in communication with a bladder, and (4) a filtrate pump with an inlet in communication with the filtration system filtrate side and an outlet in communication with the re-absorption system feed side using a computer controller in electronic communication with the filtration system inlet valve, the re-absorption system first outlet valve, the re-absorption system second outlet valve and the filtrate pump.

The computer controller can be in wireless electronic communication with the filtration system inlet valve, the re-absorption system first outlet valve, the re-absorption system second outlet valve and the filtrate pump.

The computer controller can be in electronic communication with the filtration system inlet valve, the re-absorption system first outlet valve, the re-absorption system second outlet valve and the filtrate pump using a wired connection within the percutaneous drive line.

The method can further include executing a computer readable instruction in the computer controller for operating the therapy system in a preselected functioning mode selected from a filtration mode, a re-absorption mode, a filter cleaning mode and a urine flushing mode.

The pre-selected functioning mode can be the filtration mode and executing the computer readable instruction for operating the system for closing the filtration system inlet valve, the re-absorption system first outlet valve, the re-absorption system second outlet valve while controlling the operation of the filtrate pump for pumping filtrate from the filtration system to the re-absorption system.

The pre-selected functioning mode can be the re-absorption mode and executing the computer readable instruction for operating the system for closing the filtration system inlet valve, the re-absorption system first outlet valve, the re-absorption system second outlet valve while controlling the operation of the filtrate pump for pumping filtrate through the second non-silicon ceramic filter.

The pre-selected functioning mode can be the filter cleaning mode and executing the computer readable instruction for operating the system for opening the filtration system inlet valve and the re-absorption system first outlet valve and closing the re-absorption system second outlet valve while controlling the operation of the filtrate pump for pumping a cleaning fluid.

The pre-selected functioning mode can be the urine flushing mode and executing the computer readable instruction for operating the system for closing the filtration system inlet valve and the re-absorption system first outlet valve and opening the re-absorption system second outlet valve while controlling the operation of the filtrate pump for pumping filtrate from the re-absorption system to the and the graft in communication with the bladder.

The method can include the flowing blood from a patient step which can further include executing a computer readable instruction for controlling operation of a blood pump during a pre-selected functioning mode.

The filtering step using the first non-silicon ceramic filter can provide a filtrate of blood components less than 66,500 Daltons into the filtration system filtrate side.

The filtering step using the second non-silicon ceramic filter can provide a filtrate with components within the filtrate feed side having a molecular weight of less than 500 Daltons into the re-absorption system filtrate side and into the valveless blood flow circuit.

The first non-silicon ceramic filter can be selected to maintain substantially all albumin within the blood flowing within the valveless blood flow circuit flowing from the inlet to the filtration system feed side through the outlet to the filtration system feed side while passing through to the filtration system filtrate side molecular weight fluids having a molecular weight below 65,000 Dalton.

The filtering steps through the first non-silicon ceramic filter and the second non-silicon ceramic filter can include filtering pathways formed in one or more of aluminum oxide, zinc oxide, titanium oxide, and pyrolytic carbon.

The method can further include (1) one or more of communicating a pressure reading from a sensor measuring a pressure within the filtration system to the controller using the data communication channel of the percutaneous drive line; (2) communicating a pressure reading from a sensor measuring a flow within the filtration system to the controller using the data communication channel of the percutaneous drive line; (3) communicating a pressure reading from a sensor measuring a pressure within the re-absorption system to the controller using the data communication channel of the percutaneous drive line; (4) or communicating a pressure reading from a sensor measuring a flow within the re-absorption system to the controller using the data communication channel of the percutaneous drive line.

The method can further include communicating an output of one or more of a sensor to measure a quantity of a blood protein, a sensor to measure a quantity of a blood glucose, a sensor to measure a quantity of a blood urea or a sensor to measure a quantity of a blood creatinine within the filtration system to the controller using the data communication channel of the percutaneous drive line.

The method can further include communicating an output of one or more of a sensor to indicate fouling or fracture of the non-silicon ceramic filter in the filtration system or a sensor to indicate fouling or fracture of the non-silicon ceramic filter in the re-absorption system to the controller using the data communication channel of the percutaneous drive line.

The method can further include executing computer readable instructions in the computer controller for operating the filtration system inlet valve, the re-absorption system first outlet valve, the re-absorption system second outlet valve and the filtrate pump in response to one or more communicating steps.

The implantable housing containing the components of the filtration system and the re-absorption system can be percutaneously implanted adjacent to one of an artery, a vein or a bladder.

The implantable housing containing the components of the filtration system and the re-absorption system can be subcutaneously implanted to be adjacent to a skin of the patient receiving the therapy.

The controller and a housing containing the components of the filtration system and the re-absorption system can be external to the patient and connected percutaneously to the artery graft, the vein graft and the graft connected to the bladder.

Components of the filtration system and re-absorption system can be subcutaneously-implanted with appropriate percutaneous access or subcutaneous access to a left side vein, a left side artery and a bladder or a urine collection bag or to a right side vein, a right side artery and a bladder or a urine collection bag.

The filtration and re-absorption system or associated method can provide processed filtrate or double processed filtrate.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
FIG. 1 shows the implantable renal replacement therapy device 100.
FIG. 2 is a schematic side view diagram 200 of the implanted filtration system 110.
FIG. 3 is a schematic diagram 300 of the implanted filtration system 110 with a blood pump 255.
FIG. 4 is a view 400 of the implanted filtration system 110 as in FIG. 2 configured to operate in a urine flushing mode.
FIG. 5 is a view 425 of the implanted filtration system 110 as in FIG. 2 configured to operate in a filter cleaning mode.
FIG. 6 is a schematic view 450 of the implanted filtration system 110 as in FIG. 3 configured to operate in a urine flushing mode.
FIG. 7 is a schematic view 475 of the implanted filtration system 110 as in FIG. 3 configured to operate in a filter cleaning mode.
FIG. 8 is a view of a filtration system as in FIG. 1 implanted at an appropriate left side implantation site as described above.
FIG. 9 is a therapy method 500 which describes the blood cleaning methods, techniques and mechanisms which are used in the implantable renal replacement therapy system of FIGs. 1, 2 or 3 and other configurations such as external (extracorporeal) and subcutaneously-implanted versions of the embodiments of the device.
FIG. 10 is a therapy method 600 which describes a more general form of method 500 of FIG. 9 for applications beyond renal replacement therapy.

### DETAILED DESCRIPTION

The present invention relates to renal replacement therapies, and particularly to an implantable medical device to remove uremic toxins and excess water from blood of patients with end stage renal disease and to excrete some of them through bladder. This invention also describes how to re-absorb some molecules with molecular weight <500 Da such as some salts and water back into the blood from what was filtered from blood.

The various alternative embodiments of the implantable system for renal replacement therapy may include a wide variety of different configurations providing many advantages over conventional systems. In one of more different embodiments of the inventive system may include, in any combination one of more of the following advantageous capabilities:
The use of non-silicon ceramic filter materials including a filtration system having a feed side, a filtrate side and a non-silicon ceramic filter separating the feed side and the filtrate side along with or optionally a re-absorption system having a feed side, a filtrate side and a non-silicon ceramic filter separating the feed side and the filter side. The filtration system non-silicon ceramic filter and the re-absorption system non-silicon ceramic filter may contain filtration flow paths formed from one or more of aluminum oxide, zinc oxide, titanium oxide, and pyrolytic carbon in any configuration.

At least one filter to block passage of albumin (MW=66.5 kDa) from blood under defined pressure, while letting blood components and fluids with molecular weights smaller than 66.5 kDa pass though;
At least one filter to re-absorb some molecules with molecular weight MW < 500 Da back into the blood from the filtrate of the filter(s) described in (a), while blocking the passage of some other molecules with molecular weight MW < 500 Da;
At least one pump to create and maintain defined pressure on at least one of the filters.

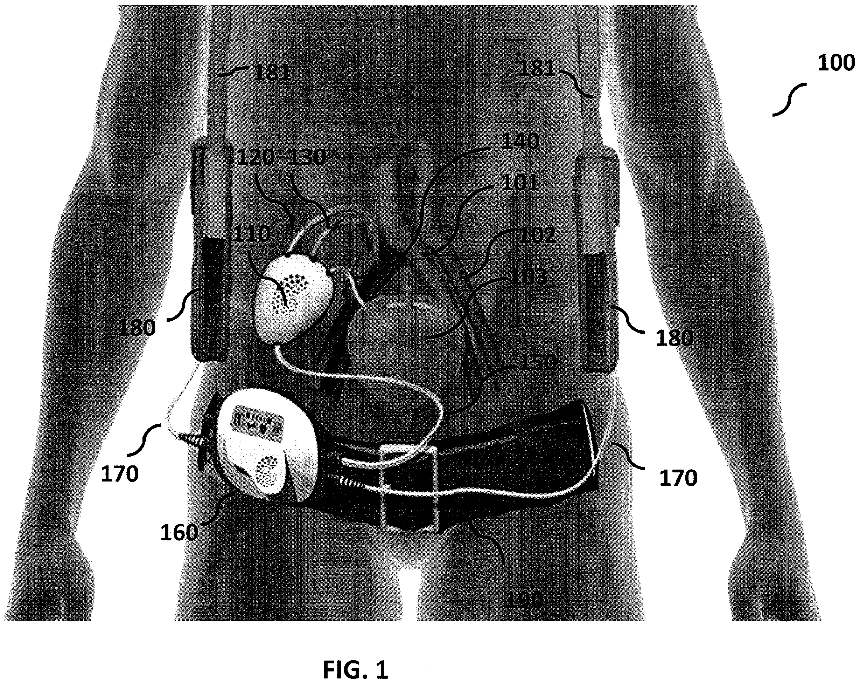

At least one cleaning mechanism to open pores and clean the surface of filters in case of fouling;
At least one optional sensor measuring quantities such as pressure, membrane fouling, membrane fracture, blood protein, blood glucose, blood urea, blood creatinine, the sensor may be placed directly in contact with body fluids or positioned for measuring the values indirectly without contact with the body fluids;
At least a power source which is a battery or a wired connection or a wireless method of transferring electric power to the device or combination of at least two of these methods;
At least one control unit;
A wired or wireless communication system to establish a line of communication between the device inside the body of the patient and a control unit outside the body of the patient, to send and receive data and to control the function of different components of the device. These features and others are better appreciated with reference to FIGs. 1-10 and the description that follows.

In some embodiments, a protein-free or substantially free ultrafiltrate generated by embodiments of the present invention may be in itself valuable and useful for ends other than the removal of toxins in blood filtering applications. For example, in various embodiments of the ultrafiltration devices of the present invention also find use in diagnostic applications. For example, the devices provides a means for selectively screening out undesired molecules (e.g., proteins) within fluids, such that a particular analyte to be analyzed (e.g., small molecules such as glucose, lactic acid, electrolytes, ions, including, but not limited to, potassium, sodium, calcium, chloride, oxygen, and carbon dioxide) in the absence of interfering molecules. Present electrochemical sensors for glucose measurement are severely hampered by protein fouling of the sensor, and great effort is devoted to the invention of fouling retardants to prolong sensor life. An ultrafiltrate substantially free of proteins, but still containing smaller constituents of blood, including but not limited to sodium, potassium, chloride, glucose, provides a solution to assay for glucose concentration without protein fouling. Thus, various embodiments of the present invention further provide systems for use in the analysis of small molecule, including, but not limited to those listed above. Furthermore, as the intracellular aqueous milieu differs from extracellular fluid, the separate testing of whole blood and a protein and cell-free ultrafiltrate for electrolyte compositions, magnetic susceptance, optical, infrared, or magnetic resonance spectroscopy, and other physical properties of matter, provides detailed information regarding the cellular composition of the blood.

Furthermore, in still another aspect a protein and cell free ultrafiltrate of blood so generated may be in itself valuable and useful for ends other than the removal of toxins and the measurement of the constituents of blood. The constituents of blood necessary for at least temporary support of a metabolically active cell are small in molecular size (including but not limited to oxygen, glucose, insulin, triiodothyronine, and retinoic acid, for example) while those immune mediators responsible for rejection of an allograft or xenograft are large in molecular size, such as antibodies, or components of the complement cascade, or reside in cell membranes, such as the major histocompatibility complexes. Thus a stream of ultrafiltrate of blood may be used to supply nutrients and carry away wastes by an efficient convective transport process, rather than by less efficient diffusive transport. As a result, various embodiments of the systems described herein may be directly applicable to any generalized cell population considered for transplantation, including but not limited to islet cell transplantation, liver cell transplantation, kidney cell transplantation, and in general transplant of any allo- or xeno- geneic cell type.

As used herein, the term "free of" refers to fluids of mixtures that have had one or more components (e.g., protein components) removed. "Substantially free of" fluids or mixtures are at least 50% free, preferably at least 75% free, and more preferably at least 90% free from a component with which they are otherwise naturally associated. For example, a fluid that is "substantially free of protein" is a fluid that has at least 50% or less of the protein content of an unfiltered or unpurified fluid.

As used herein, the term "dialysis" refers to a form of filtration, or a process of selective diffusion through a membrane; it is typically used to separate low-molecular weight solutes that diffuse through the membrane from the colloidal and high-molecular weight solutes which do not. In some embodiments, a feed of fluid is passed over a semipermeable membrane, and a feed of dialysate is passed over the other side of that membrane; the membrane is wetted by one or both solvents, and then there is diffusive transport of dissolved solutes between the fluids. The composition of one fluid, the dialysate, is used to deplete the composition of the other fluid, the feed fluid, of some molecule or molecules.

As used herein, the term "filtration" refers to a process of separating particulate matter from a fluid, such as air or a liquid, by passing the fluid carrier through a medium that will not pass the particulates.

As used herein, the term "ultrafiltration" refers to subjecting a fluid to filtration, where the filtered material is very small; typically, the fluid comprises colloidal, dissolved solutes or very fine solid materials, and the filter is a microporous, nanoporous, or semipermeable medium. A typical medium is a membrane. The fluid to be filtered is referred to as the "feed fluid." During ultrafiltration, the feed fluid is separated into a "permeate" or "filtrate" or "ultrafiltrate," which has been filtered through the medium, and a "retentate," which is that part of the feed fluid which did not get filtered through the medium, or which is retained by the medium.

FIG. 1 shows the implantable renal replacement therapy device 100.

The implanted filtration system 110 is connected to the control unit 160 through percutaneous driveline 150.

The implanted filtration system 110 is connected to an artery (such as iliac artery) 102 through the tube 120. The implanted filtration system 110 is connected to iliac vein 101 (or any other vein) through the tube 130.

The implanted filtration system 110 is connected to bladder 103 through the tube 140.

The tubes 120, 130 and 140 can be medical grade polymer grafts.

The implanted filtration system 110 has at least one filtration system to remove some blood components smaller than 65,000 Da in molecular weight (such as uremic toxins, salts and water) from blood.

The implanted filtration system 110 may have at least one re-absorption system to return some blood components with molecular weight smaller than 500 Daltons (such as some salt and water) back into the blood from the filtrate generated by the filtration system.

The percutaneous driveline 150 contains power cords and fluid transfer tubes and data transfer lines and connects the control unit 160 to the implanted filtration system 110. The control unit 160 is connected to the batteries 180 through power cords 170. The batteries can be placed in hangers 181. Additionally, the control unit 160 is attached to the belt 190.

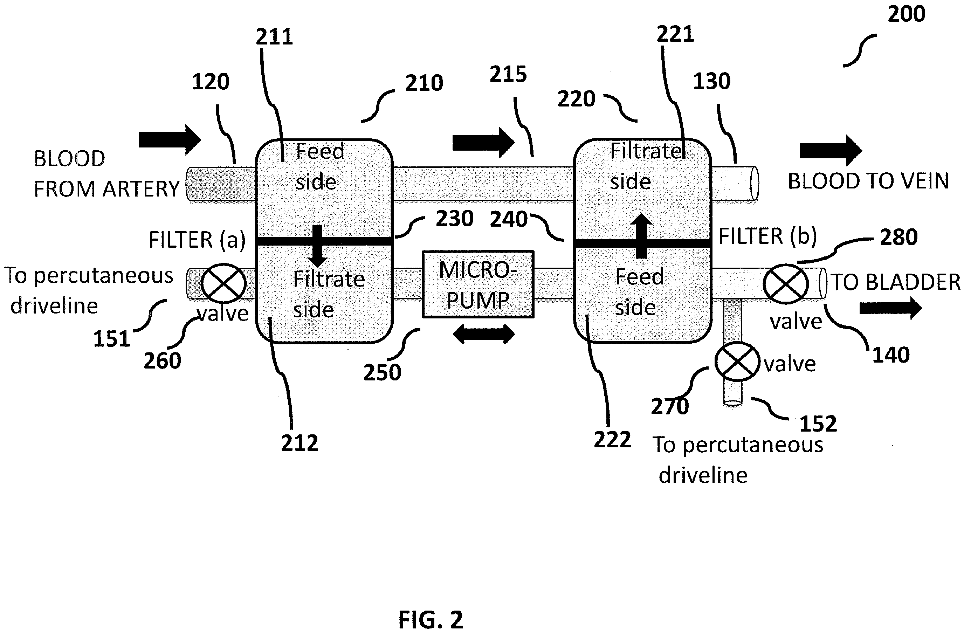

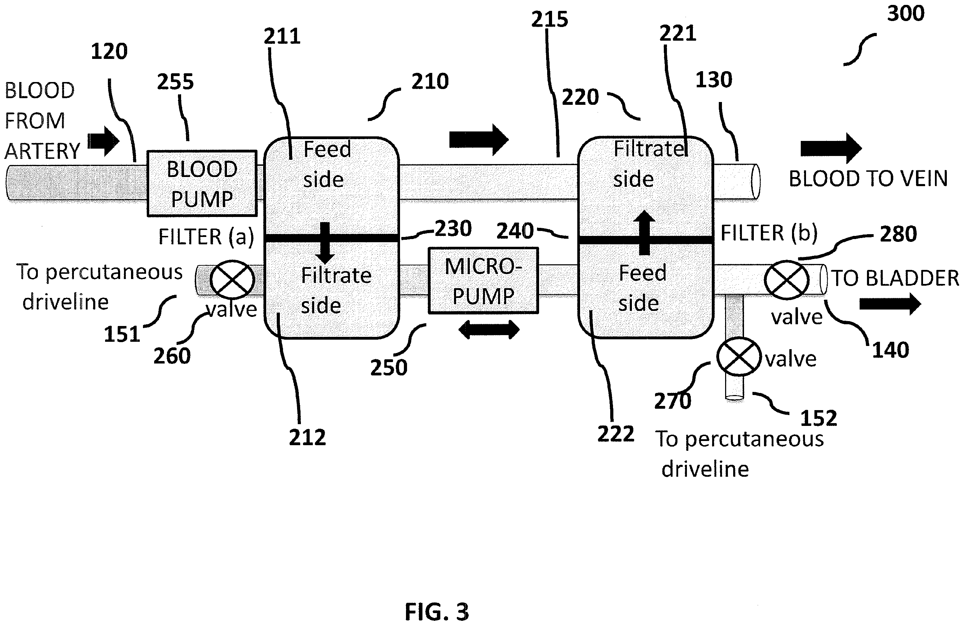

The control unit 160 includes an electronic display (i.e., an LCD or a touch screen) and a computer controller in electronic communication with the components of the filtration system and the re-absorption system. The control unit 160 is configured to operate or control, for example, the filtration system inlet valve, the re-absorption system first outlet valve, the re-absorption system second outlet valve and the filtrate pump as in FIG. 2 as well as a blood pump when included as in FIG. 3. Tables 1 and 2 below provide exemplary configurations of these valves for operating the filtration and re-absorption system in a preselected functioning mode. By way of illustration and not limitation, the pre-selected functioning modes may be selected from a filtration mode, a re-absorption mode, a filter cleaning mode, a urine flushing mode and a service mode.

The fluid transfer tubes in the percutaneous driveline 150 are mainly used to transfer fluids into and out of the implanted filtration system 110 to wash the filters and to open the clogged pores of the filters. The washing solution can be at least one of intravenous (IV) fluid, saline solution, dialysate or replacement fluid used in renal replacement therapy or any other fluid that is safe to blood if it passes through the filters into the blood.

At least one bag of washing solution can be attached to the control unit 160. The control unit 160 can pump the washing solution into the implanted filtration system 110 through the percutaneous driveline 150. The control unit 160 can collect the used washing solution in the same or another bag from the implanted filtration system 110 through the percutaneous driveline 150.

The tubes 120, 130 and 140 are manufactured from bio-compatible materials such as medical grade Dacron or medical grade Polytetrafluoroethylene (PTFE).

FIG. 2 is a schematic side view diagram 200 of the implanted filtration system 110.

Blood is diverted from an artery through the connection 120 into the filtration system 210.

In one embodiment, the blood flow rate at the device inlet is up to 200 ml/min which is set by the size of the graft 120.

Blood is passed across the feed side 211 of at least one filter 230 in the filtration system 210. The filtration system 210 has at least one filter to remove some blood components smaller than 65,000 Da in molecular weight (such as uremic toxins, salts and water) from blood. The filtrate is collected at the filtrate side 212 of the filter 230 and is pumped into the feed side 222 of the re-absorption system 220 using a micro-pump 250 when the valves 260, 270 and 280 are closed. The micro-pump 250 creates a pressure on one side 222 of the filter 240 which is higher than the pressure at the other side 221 of the filter 240. Blood is passed across one side 221 of at least one filter 240 in the re-absorption system 220. The re-absorption system 220 has at least one filter to return some blood components with molecular weight smaller than 500 Daltons (mainly some salt and water) back into the blood from the filtrate generated by the filtration system 210.

In another embodiment, the re-absorption system 220 has at least one filter to return some blood components with molecular weight smaller than 66,500 Daltons back into the blood from the filtrate generated by the filtration system 210.

In another embodiment, the re-absorption system 220 has at least one filter to return some blood components with molecular weight smaller than 70,000 Daltons back into the blood from the filtrate generated by the filtration system 210.

In another embodiment, the re-absorption system 220 has at least one filter to return some blood components with molecular weight smaller than 200,000 Daltons back into the blood from the filtrate generated by the filtration system 210. In other embodiments, the pore size or filtration capacity of the filtration system and the pore size or filtration capacity of the re-absorption system many vary depending upon desired operating characteristics. In some aspects, one or more different or additional filters or other additional processing components may be included within the implantable filtration unit 110, the control unit 160, an embodiment of a filtration system or an embodiment of a re-absorption system or as part of a configuration within one or both of the filtration system or the re-absorption system to provide "processed filtrate" or "double processed filtrate" capabilities as described herein.

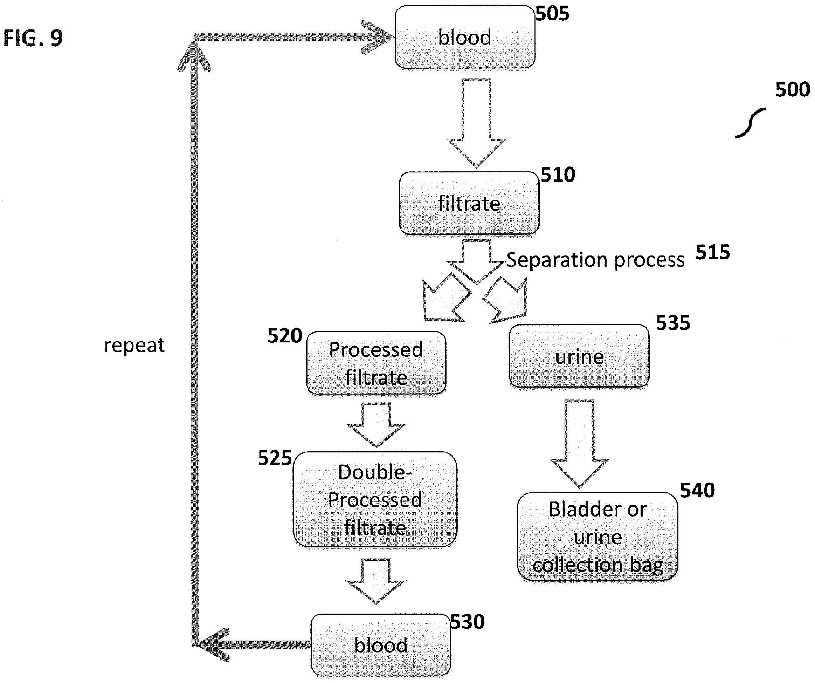

In another embodiment, one of or the combination of filtrate side 212, feed side 222, micro-pump 250 and re-absorption system 220 produce(s) at least one of "processed filtrate" and "double processed filtrate" as described below in method 500 (FIG. 9).

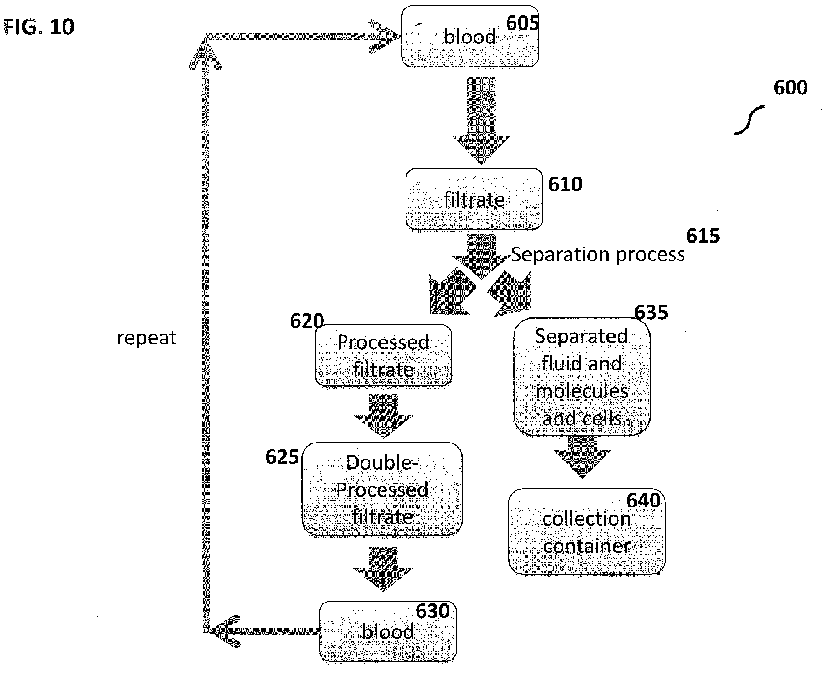

In another embodiment, one of or the combination of filtrate side 212, feed side 222, micro-pump 250 and re-absorption system 220 produce(s) at least one of "processed filtrate" and "double processed filtrate" as described below in method 600 (FIG. 10).

Tube 215 connects filtration system 210 to the re-absorption system 220.

Blood from the filtration system 210 goes into the re-absorption system 220 through the connection tube 215.

Tube 130 connects the re-absorption system 220 to the vein.

Blood from the re-absorption system 220 is passed into the vein 101 through the tube 130.

Blood flows from the tube 120 to the tube 130 due to the higher blood pressure in the artery 102 in comparison to the blood pressure in the vein 101.

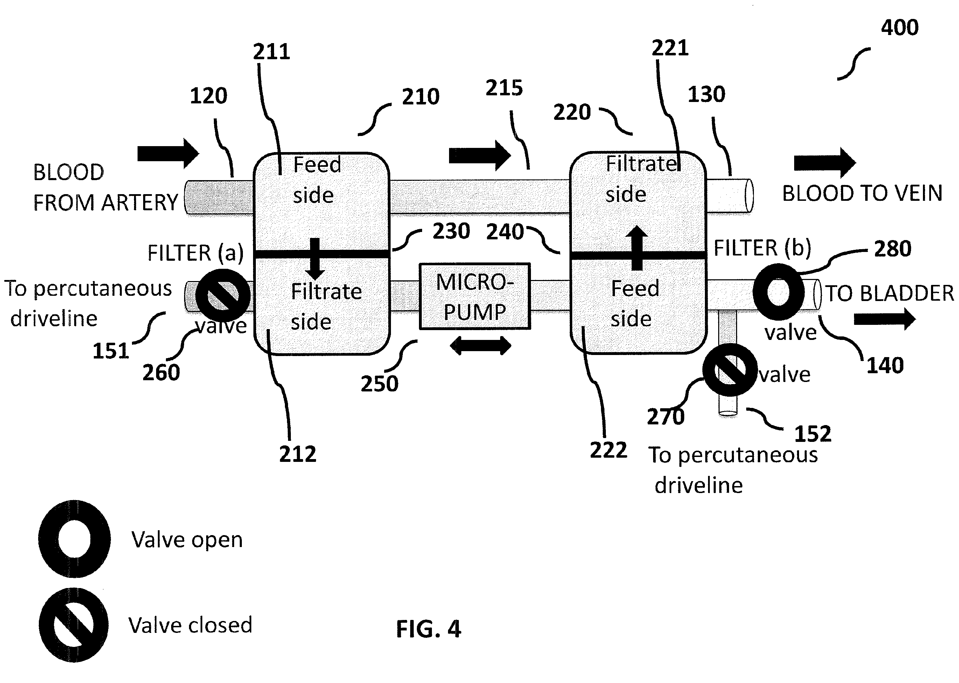

FIG. 4 is a view 400 of the implanted filtration system 110 as in FIG. 2 configured to operate in a urine flushing mode.

When valve 260 and valve 270 are closed and valve 280 is open, the micro-pump 250 can be used to transfer fluids from the 212 side of the filtration system 210 and the 222 side of the re-absorption system 220 to bladder 103 through the tube 140.

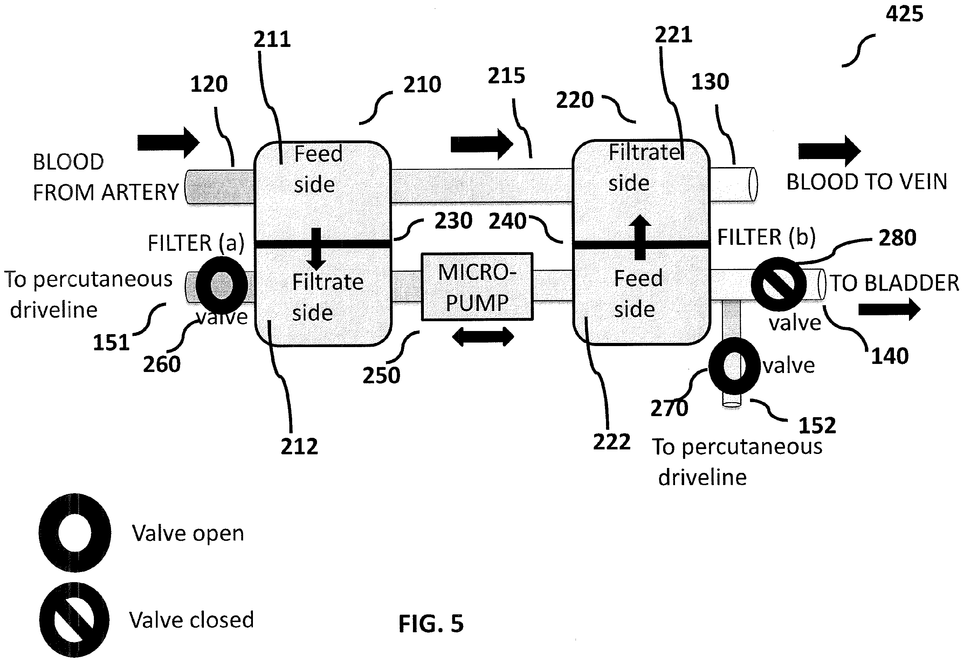

FIG. 5 is a view 425 of the implanted filtration system 110 as in FIG. 2 configured to operate in a filter cleaning mode.

When valve 280 is closed and valve 260 and valve 270 are open, at least one washing solution can be pumped from percutaneous driveline into and out of the implanted filtration system 110 through the connection tubes 151 and 152. The washing solution can minimize the membrane fouling and pore clogging in the filters 230 and 240.

FIG. 3 is a schematic diagram 300 of the implanted filtration system 110 with a blood pump 255.

Blood is diverted from an artery through the connection 120 and is pumped into the filtration system 210. In one embodiment, the blood flow rate at the device inlet is up to 200 ml/min which is set by the blood pump 255. In another embodiment, blood flow > 500 ml /min, preferably > 800 ml/min.

Blood is passed across the feed side 211 of at least one filter 230 in the filtration system 210. The filtration system 210 has at least one filter to remove some blood components smaller than 65,000 Da in molecular weight (such as uremic toxins, salts and water) from blood. The filtrate is collected at the filtrate side 212 of the filter 230 and is pumped into the feed side 222 of the re-absorption system 220 using a micro-pump 250 when the valves 260, 270 and 280 are closed. The micro-pump 250 creates a pressure on one side 222 of the filter 240 which is higher than the pressure at the other side 221 of the filter 240. Blood is passed across one side 221 of at least one filter 240 in the re-absorption system 220. The re-absorption system 220 has at least one filter to return some blood components with molecular weight smaller than 500 Daltons (mainly some salt and water) back into the blood from the filtrate generated by the filtration system 210.

In another embodiment, the re-absorption system 220 has at least one filter to return some blood components with molecular weight smaller than 66,500 Daltons back into the blood from the filtrate generated by the filtration system 210.

In another embodiment, the re-absorption system 220 has at least one filter to return some blood components with molecular weight smaller than 70,000 Daltons back into the blood from the filtrate generated by the filtration system 210.

In another embodiment, the re-absorption system 220 has at least one filter to return some blood components with molecular weight smaller than 200,000 Daltons back into the blood from the filtrate generated by the filtration system 210.

In another embodiment, one of or the combination of filtrate side 212, feed side 222, micro-pump 250 and re-absorption system 220 produce(s) at least one of "processed filtrate" and "double processed filtrate" as described in schematic 500.

In another embodiment, one of or the combination of filtrate side 212, feed side 222, micro-pump 250 and re-absorption system 220 produce(s) at least one of "processed filtrate" and "double processed filtrate" as described in schematic 600.

Tube 215 connects filtration system 210 to the re-absorption system 220.

Tube 130 connects the re-absorption system 220 to the vein.

Blood from the re-absorption system 220 is passed into the vein 101 through the tube 130.

Blood flows from the tube 120 to the tube 130 due to the pressure created by the blood pump 255 and higher blood pressure in the artery 102 in comparison to the blood pressure in the vein 101.

The pressure created by the blood pump 255 pushes the blood from the filtration system 210 into the re-absorption system 220 through the connection tube 215.

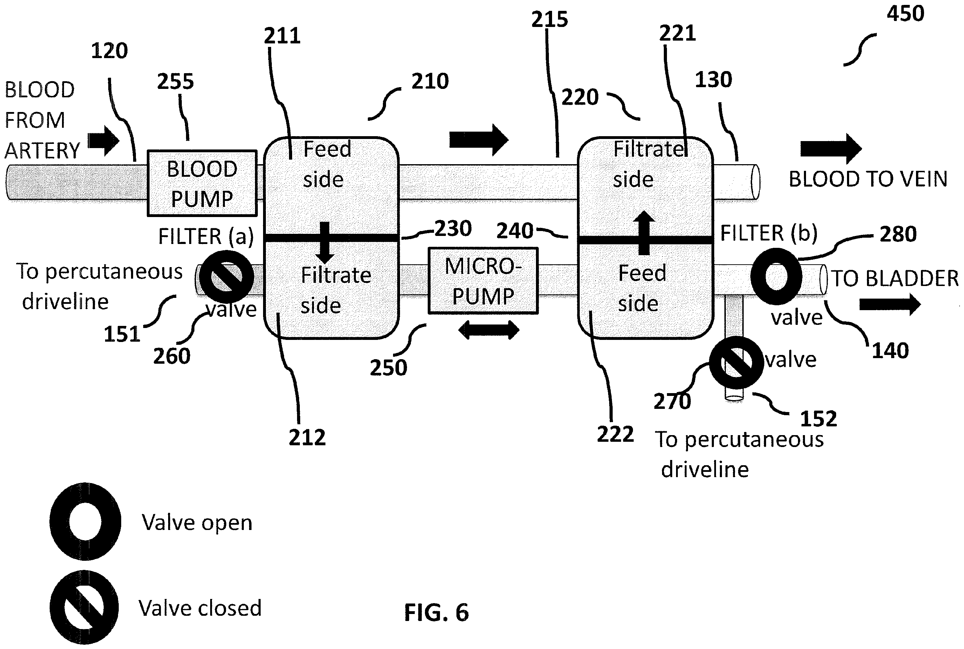

FIG. 6 is a schematic view 450 of the implanted filtration system 110 as in FIG. 3 configured to operate in a urine flushing mode.

When valve 260 and valve 270 are closed and valve 280 is open, the micro-pump 250 can be used to transfer fluids from the 212 side of the filtration system 210 and the 222 side of the re-absorption system 220 to bladder 103 through the tube 140.

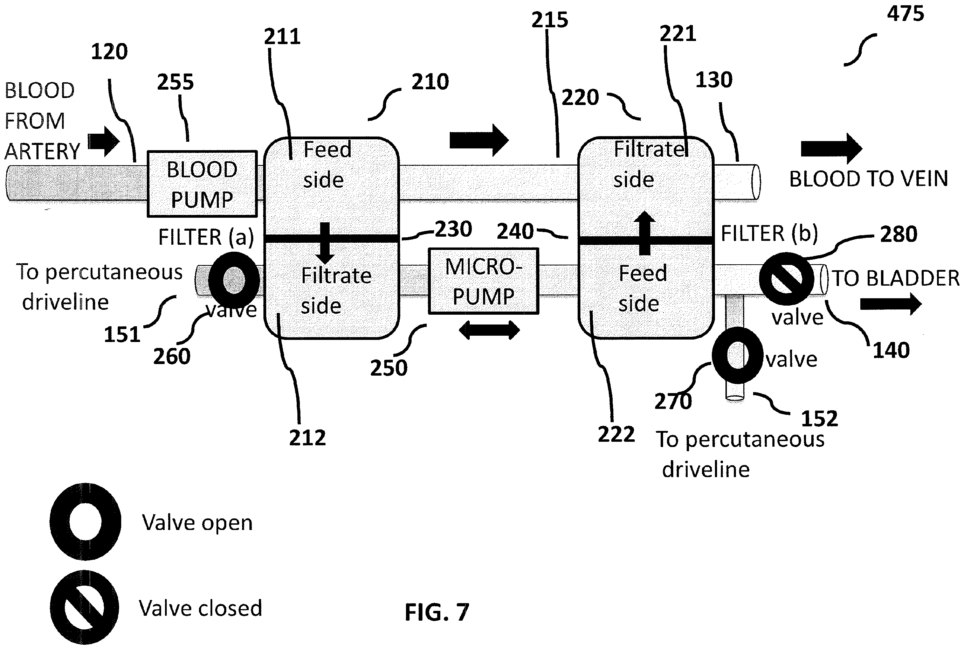

FIG. 7 is a schematic view 475 of the implanted filtration system 110 as in FIG. 3 configured to operate in a filter cleaning mode.

When valve 280 is closed and valve 260 and valve 270 are open, at least one washing solution can be pumped from percutaneous driveline into and out of the implanted filtration system 110 through the connection tubes 151 and 152. The washing solution can minimize the membrane fouling and pore clogging in the filters 230 and 240.

Table 1 describes various functioning modes and corresponding state of the device 200 of FIG. 2 which is set by the controller 160.

**Table 1**

| | Valve 260 | Valve 270 | Valve 280 | Micro-pump 250 |
|---|---|---|---|---|
| Filtration mode | closed | closed | closed | off/on |
| Re-absorption mode | closed | closed | closed | on |
| Filter cleaning mode | open | open | closed | on |
| Urine flushing mode | closed | closed | open | on |

Table 2 describes various functioning modes and corresponding state of the device 300 of FIG. 3 which is set by the controller 160.

**Table 2**

| | Valve 260 | Valve 270 | Valve 280 | Micro-pump 250 | Blood pump 255 |
|---|---|---|---|---|---|
| Filtration mode | closed | closed | closed | off/on | on |
| Re-absorption mode | closed | closed | closed | on | on |
| Filter cleaning mode | open | open | closed | on | on |
| Urine flushing mode | closed | closed | open | on | on |

In some embodiments, a pump operating within the implantable filtration unit 110, a filtration system or a re-absorption system may be operated in a forward or reverse direction depending upon desired operations and fluid movement. As such, Table 1 and Table 2 may also indicate "on" but also direction (forward or reverse) as well as stead state, pulsed, increasing speed or decreasing speed based on operational mode and control systems and instructions within the control unit. Moreover, while some embodiments of the filtration system have been illustrated and described as having an external controller with implanted filtration systems 200, 300, other configurations are possible.

In one additional embodiment, the control unit 160 and single pump filtration system 200 (the system of FIGs. 1 and 2) be outside of the body with appropriate percutaneous access or subcutaneous access to a left side vein, a left side artery and a urine collection bag or to a right side vein, a right side artery and a urine collection bag.

In still another additional embodiment, the control unit 160 and dual pump filtration system 300 (the system of FIGs. 1 and 3) be outside of the body with appropriate percutaneous access or subcutaneous access to a left side vein, a left side artery and a urine collection bag or to a right side vein, a right side artery and a urine collection bag.

In still another additional embodiment, the control unit 160 be outside of the body with single pump filtration system 200 (the system of FIG. 2) subcutaneously-implanted with appropriate percutaneous access or subcutaneous access to a left side vein, a left side artery and a bladder or urine collection bag or to a right side vein, a right side artery and a bladder or a urine collection bag.

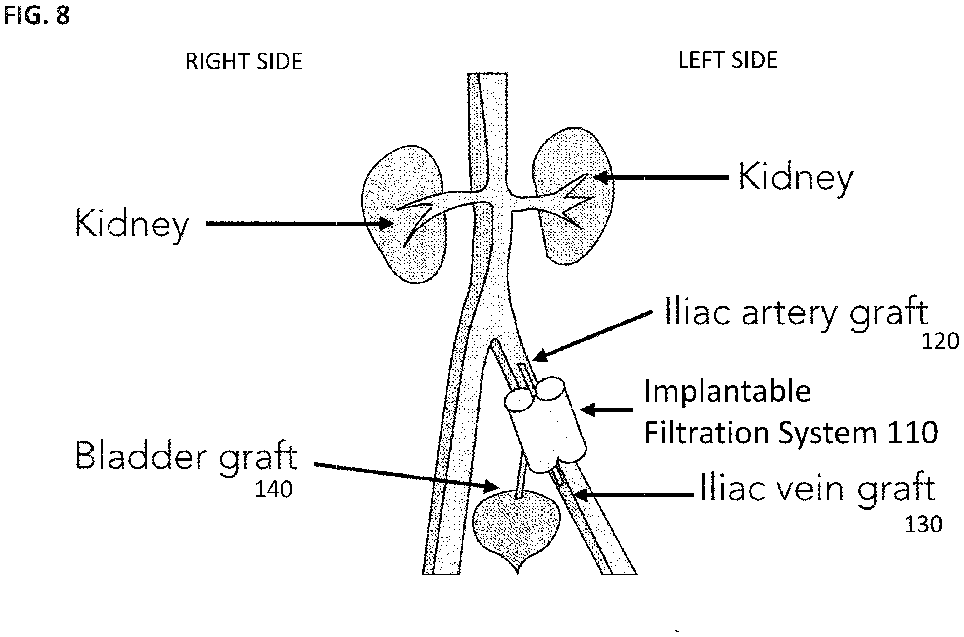

In still another additional embodiment, the control unit 160 be outside of the body with dual pump filtration system 300 (the system of FIG. 3) subcutaneously-implanted with appropriate percutaneous access or subcutaneous access to a left side vein, a left side artery and a bladder or a urine collection bag or to a right side vein, a right side artery and a bladder or a urine collection bag. FIG. 8 is a view of a filtration system as in FIG. 1 implanted at an appropriate left side implantation site as described above.

In combination and with modifications according to the various configurations above, in one aspect, the computer controller includes a computer readable instruction for operating the system in a preselected functioning mode selected from a filtration mode, a re-absorption mode, a filter cleaning mode, a urine flushing mode and a service mode.

In one specific embodiment, the pre-selected functioning mode is the filtration mode with the computer readable instruction for operating the system further comprising instructions to close the filtration system inlet valve, the re-absorption system first outlet valve, the re-absorption system second outlet valve while controlling the operation of the filtrate pump to pump filtrate from the filtration system to the re-absorption system.

In one specific embodiment, the pre-selected functioning mode is the re-absorption mode with the computer readable instruction for operating the system further comprising instructions to close the filtration system inlet valve, the re-absorption system first outlet valve, the re-absorption system second outlet valve while controlling the operation of the filtrate pump to pump filtrate through the re-absorption system non-silicon ceramic filter.

In one specific embodiment, the pre-selected functioning mode is the filter cleaning mode with the computer readable instruction for operating the system further comprising instructions to open the filtration system inlet valve, open the re-absorption system first outlet valve, close the re-absorption system second outlet valve while controlling the operation of the filtrate pump to pump a cleaning fluid.

In one specific embodiment, the pre-selected functioning mode is the urine flushing mode with the computer readable instruction for operating the system further comprising instructions to close the filtration system inlet valve, close the re-absorption system first outlet valve, open the re-absorption system second outlet valve while controlling the operation of the filtrate pump to pump filtrate from the re-absorption system to the and the graft in communication with the bladder.

In one specific embodiment, the pre-selected functioning mode is the service mode with the computer readable instruction for operating the system further comprising instructions to open or close the filtration system inlet valve, open or close the re-absorption system first outlet valve, open or close the re-absorption system second outlet valve while controlling the operation of the filtrate pump to pump or not to pump the filtrate from the re-absorption system to the and the graft in communication with the bladder.

In still another embodiment in conjunction with any of the above modes the computer readable instruction for operating the system also includes controlling operation of the blood pump during a pre-selected functioning mode for systems configured as in FIG. 3.

It is to be appreciated that the various embodiments of the systems described herein may be provided in a number of different configurations to provide numerous advantageous results. In one such aspect, FIG. 9 is a therapy method 500 which describes the blood cleaning methods, techniques and mechanisms which are used in the implantable renal replacement therapy system of FIGs. 1, 2 or 3 and other configurations such as external (extracorporeal) and subcutaneously-implanted versions of the embodiments of the device.

### (i) Separation of filtrate from blood

In the first step 505 blood flows into the filtration system, some blood components are separated from blood in the form of a fluid called the filtrate (step 510). The operating characteristics of the filtration system are selected such that filtrate does not contain any platelets or is substantially free of platelets. Coagulation and thrombosis are primarily a function of endothelial cells, platelets, and soluble coagulation factors, therefore in the absence of platelets, the filtrate does not clot, even if the soluble coagulation factor would be present. This advantage allows us to further process the filtrate without the risk of coagulation and thrombosis.

In one embodiment, the filtrate is substantially free of proteins (such as albumin) and cellular elements (such as red blood cells, white blood cells and platelets).

In one additional embodiment, the filtrate may still have substantial amount of proteins (such as albumin), but is substantially free of cellular elements (such as red blood cells, white blood cells and platelets).

### (ii) Filtrate processing stage 1

The collected filtrate is processed in this step.

For renal replacement therapy application, uremic toxins, excess fluid and excess solutes are separated from the filtrate at step 515. The combination of the removed uremic toxins, excess fluid and excess solutes makes urine (step 535). "Processed filtrate" in step 520 refers to the filtrate remains after removal of uremic toxins, excess fluid and excess solutes. In one embodiment, the "processed filtrate" is substantially free of at least one of water soluble uremic toxins, protein bound uremic toxins, proteins (such as albumin), excess fluids, excess solutes. In another embodiment, this step can be done using at least one of filter 230 in device 200. In still another embodiment, this step can be done using filter 230 in device 300.

### (iii) Filtrate processing stage 2

"Processed filtrate" is processed again at step 525 to make it chemically, biologically and physically suitable and safe to be returned to the blood. Processed filtrate that undergoes such additional processing is "double processed filtrate".

For example, in one embodiment, one or all of the temperature, osmolality and pH of the "processed filtrate" are adjusted according to requirements set for the patient.

### (iv) Urine removal:

Urine is removed from the system and is collected in a bag or in bladder as in step 540.

### (v) re-absorption process.

The "double processed filtrate" is returned to the blood safely as in step 530.

In one embodiment, up to 99.5 % of the filtrate collected in stage (i) may be returned back to the blood in this stage.
(vi) Blood is circulated in the body of the patient and the steps (i) to (vi) (steps 505 through 530) are repeated until the set outcome is achieved.

In still further alternatives are appreciated with reference to FIG. 10. FIG. 10 is a therapy method 600 which describes a more general form of method 500 of FIG. 9 for applications beyond renal replacement therapy.

### (i) Separation of filtrate from blood

In the first step 605, some blood components entering the filtration system are separated from blood in the form of a fluid called the filtrate (step 610). The operating characteristics of the filtration system as selected such that filtrate does not contain any platelets or is substantially free of platelets. Coagulation and thrombosis are primarily a function of endothelial cells, platelets, and soluble coagulation factors, therefore in the absence of platelets, the filtrate does not clot, even if the soluble coagulation factor would be present. This advantage allows us to further process the filtrate without the risk of coagulation and thrombosis.

In one embodiment, the filtrate is substantially free of proteins (such as albumin) and cellular elements (such as red blood cells, white blood cells and platelets).

In an additional embodiment, the filtrate may still have substantial amount of proteins (such as albumin), but is substantially free of cellular elements.

In an additional embodiment, the filtrate may still have substantial amount of proteins (such as albumin), but is substantially free of at least one of red blood cells, white blood cells and platelets.

### (ii) Filtrate processing stage 1

The collected filtrate from step 610 is processed in this step. Targeted blood components, molecules or cells or what is circulating in the blood stream are separated in the separation process of step 615.

In one embodiment circulating tumor cells are separated from the collected filtrate.

In another embodiment pathogens such as bacteria and viruses can be separated from the collected filtrate.

### (iii) Filtrate processing stage 2

"Processed filtrate" from step 620 is processed again to make it chemically, biologically and physically suitable and safe to be returned to the blood. We call the result in step 625 the "double processed filtrate".

For example, in one embodiment, one or all of the temperature, osmolality and pH of the "processed filtrate" are adjusted according to requirements set for the patient.

### (iv) Removal of separated fluid and molecules and cells:

At least one of the fluid and molecules and cells which were separated in stage (ii) step 635 are collected in a container at step 640.

### (v) re-absorption process.

The "double processed filtrate" from step 625 is returned to the blood safely at step 630.
(vi) Blood is circulated in the body of the patient and the steps (i) to (vi) are repeated until the set outcome is achieved.

Additional details for various additional alternative or additional embodiments may be appreciated by reference to US Patent Application Publication US 2012/0289881, US Patent Application Publication US 2016/0332119, US Patent 8682431, US Patent 9452249, US Patent Application US20160064117, US Patent Application US20160181730 US Patent 8827890, US Patent 7998054, US Patent Application US20120022645, US Patent Application US20150290377 WIPO Patent Application WO2015134871, US Patent Application US20150290378, US Patent Application US20150290374 , US Patent Application US20150294550 and US Patent Application US20160175502.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative embodiments are described above, any of a number of changes may be made to various embodiments without departing from the scope of the invention as described by the claims. For example, the order in which various described method steps are performed may often be changed in alternative embodiments, and in other alternative embodiments one or more method steps may be skipped altogether. Optional features of various device and system embodiments may be included in some embodiments and not in others.

## Claims

1. A renal replacement therapy system, comprising:
A filtration system (210) having a feed side (211), a filtrate side (212) and a non-silicon ceramic filter (230) separating the feed side and the filtrate side;
A re-absorption system (220) having a feed side (222), a filtrate side (221) and a non-silicon ceramic filter (240) separating the feed side and the filtrate side;
A valveless blood flow circuit in communication with an arterial graft, an inlet to the filtration system feed side, an outlet to the filtration system feed side, an inlet to the re-absorption system filtrate side, an outlet to the re-absorption system filtrate side and a venous graft;
A percutaneous drive line (150) including a fluid supply channel, a fluid return channel, a power cable, and a data communications cable;
A filtration system inlet valve in communication with the fluid supply channel and an inlet to the filtration system filtrate side;
A re-absorption system first outlet valve in communication with the re-absorption system feed side and the fluid return channel;
A re-absorption system second outlet valve in communication with the re-absorption system feed side and a graft in communication with a bladder (103) or a urine collection bag;
A filtrate pump (250) with an inlet in communication with the filtration system filtrate side and an outlet in communication with the re-absorption system feed side; and
An implantable housing containing the filtration system (210), the re-absorption system (220), and the filtrate pump (250).

2. The system of claim 1 wherein the implantable housing is completely sealed except for four openings.

3. The system of claim 2 wherein the four openings are positioned to provide communication to an interior portion of the implantable housing for the percutaneous drive line, the blood flow circuit inlet to the filtration system feed side (211); the blood flow circuit outlet to the re-absorption system filtrate side (221) and an opening in communication with the re-absorption system second outlet valve.

4. The system of claim 1 wherein the filtration system non-silicon ceramic filter (230) is selected to filter blood components less than 66,500 Daltons into the filtration system filtrate side (212).

5. The system of claim 1 wherein the re-absorption system non-silicon ceramic filter (240) is sized to pass components within the filtrate feed side (211) having a molecular weight of less than 500 Daltons into the re-absorption system filtrate side (221) and into the valveless blood flow circuit.

6. The system of claim 1 wherein the filtration system non-silicon ceramic filter (230) is selected to maintain substantially all albumin within the blood flowing within the valveless blood flow circuit flowing from the inlet to the filtration system feed side through the outlet to the filtration system feed side while passing through to the filtration system filtrate side (212) molecular weight fluids having a molecular weight below 66,500 Dalton.

7. The system of any of the above claims wherein the filtration system non-silicon ceramic filter (230) and/or the re-absorption system non-silicon ceramic filter (240) contains one or more of aluminum oxide, zinc oxide, titanium oxide, and pyrolytic carbon.

8. The system of any of the above claims wherein the filtration system filter (230) contains one or more of the materials and structures with fracture toughness **K_{IC}** values of higher than **1.00 (MPa . m^{1/2})**

9. The system of claim 1 further comprising one or more of a sensor to measure a pressure within the filtration system (210); a sensor to measure a flow within the filtration system; a sensor to measure a pressure within the re-absorption system (220); or a sensor to measure a flow within the re-absorption system.

10. The system of claim 1 further comprising one or more of a sensor to measure a quantity of a blood protein, a sensor to measure a quantity of a blood glucose, a sensor to measure a quantity of a blood urea or a sensor to measure a quantity of a blood creatinine within the filtration system (210) or the re-absorption system (220).

11. The system of any of the above claims further comprising a sensor to indicate fouling or fracture of the non-silicon ceramic filter in the filtration system or a sensor to indicate fouling or fracture of the non-silicon ceramic filter in the re-absorption system.

12. The system of any of the above claims further comprising: a control unit (160) with an electronic display and a computer controller in electronic communication with the filtration system inlet valve, the re-absorption system first outlet valve, the re-absorption system second outlet valve and the filtrate pump (250).

13. The system of claim 12, wherein the computer controller is in electronic communication with one or more of the sensors of claims 9-11.

14. The system of any of the above claims further comprising a blood pump with an inlet in communication with the arterial graft and an outlet in communication with the inlet to the filtration system feed side.

15. The system of any of the above claims further comprising at least one battery pack connected to the control unit (160).

## Patentansprüche

1. Nierenersatztherapiesystem, das Folgendes umfasst:
ein Filtrationssystem (210) mit einer Eingabeseite (211), einer Filtratseite (212) und einem Nicht-Silizium-Keramikfilter (230), der die Eingabeseite und die Filtratseite trennt;
ein Reabsorptionssystem (220) mit einer Eingabeseite (222), einer Filtratseite (221) und einem Nicht-Silizium-Keramikfilter (240), der die Eingabeseite und die Filtratseite trennt;
einen ventillosen Blutflusskreislauf in Verbindung mit einem Arterientransplantat, einem Einlass auf der Eingabeseite des Filtrationssystems, einem Auslass auf der Eingabeseite des Filtrationssystems, einem Einlass auf der Filtratseite des Reabsorptionssystems, einem Auslass auf der Filtratseite des Reabsorptionssystems und einem Venentransplantat;
eine perkutane Antriebsleitung (150) mit einem Fluidzufuhrkanal, einem Fluidrückführkanal, einem Stromkabel und einem Datenkommunikationskabel;
ein Filtrationssystem-Einlassventil in Verbindung mit dem Fluidzufuhrkanal und einem Einlass auf der Filtratseite des Filtrationssystems;
ein erstes Auslassventil des Reabsorptionssystems in Verbindung mit der Eingabeseite des Reabsorptionssystems und dem Fluidrückführkanal;
ein zweites Auslassventil des Reabsorptionssystems in Verbindung mit der Eingabeseite des Reabsorptionssystems und einem Transplantat, das in Verbindung mit einer Blase (103) oder einem Urinsammelbeutel steht;
eine Filtratpumpe (250) mit einem Einlass in Verbindung mit der Filtratseite des Filtrationssystems und einem Auslass in Verbindung mit der Eingabeseite des Reabsorptionssystems; und
ein implantierbares Gehäuse, das das Filtrationssystem (210), das Reabsorptionssystem (220) und die Filtratpumpe (250) enthält.

2. System nach Anspruch 1, wobei das implantierbare Gehäuse bis auf vier Öffnungen vollständig abgedichtet ist.

3. System nach Anspruch 2, wobei die vier Öffnungen so positioniert sind, dass sie eine Verbindung mit einem inneren Abschnitt des implantierbaren Gehäuses für die perkutane Antriebsleitung, den Einlass des Blutflusskreislaufs auf der Eingabeseite (211) des Filtrationssystems, den Auslass des Blutflusskreislaufs auf der Filtratseite (221) des Reabsorptionssystems und eine Öffnung in Verbindung mit dem zweiten Auslassventil des Reabsorptionssystems bereitstellen.

4. System nach Anspruch 1, wobei der Nicht-Silizium-Keramikfilter (230) des Filtrationssystems so ausgewählt ist, dass er Blutkomponenten mit weniger als 66.500 Dalton in die Filtratseite (212) des Filtrationssystems filtriert.

5. System nach Anspruch 1, wobei der Nicht-Silizium-Keramikfilter (240) des Reabsorptionssystems so bemessen ist, dass er Komponenten innerhalb der Filtrateingabeseite (211) mit einer Molekülmasse von weniger als 500 Dalton in die Filtratseite (221) des Reabsorptionssystems und in den ventillosen Blutflusskreislauf weitergibt.

6. System nach Anspruch 1, wobei der Nicht-Silizium-Keramikfilter (230) des Filtrationssystems so ausgewählt ist, dass er im Wesentlichen das gesamte Albumin innerhalb des Blutes beibehält, das im ventillosen Blutflusskreislauf fließt, der vom Einlass auf der Eingabeseite des Filtrationssystems durch den Auslass auf der Eingabeseite des Filtratsionssystems läuft, während er Fluids mit einer Molekülmasse von weniger als 66.500 Dalton zur Filtratseite (212) des Filtrationssystems durchlässt.

7. System nach einem der obigen Ansprüche, wobei der Nicht-Silizium-Keramikfilter (230) des Filtrationssystems und/oder der Nicht-Silizium-Keramikfilter (240) des Reabsorptionssystems eines oder mehrere aus Aluminiumoxid, Zinkoxid, Titanoxid und pyrolytischem Kohlenstoff enthält.

8. System nach einem der obigen Ansprüche, wobei der Filter (230) des Filtrationssystems ein oder mehrere der Materialien und Strukturen mit Bruchzähigkeits-K_{IC}-Werten von mehr als 1,00 (MPa . m^{1/2}) enthält.

9. System nach Anspruch 1, das ferner eines oder mehrere aus einem Sensor zum Messen eines Drucks innerhalb des Filtrationssystems (210); einem Sensor zum Messen eines Flusses innerhalb des Filtrationssystems; einem Sensor zum Messen eines Drucks innerhalb des Reabsorptionssystems (220); oder einem Sensor zum Messen eines Flusses innerhalb des Reabsorptionssystems umfasst.

10. System nach Anspruch 1, das ferner eines oder mehrere aus einem Sensor zum Messen einer Menge eines Blutproteins, einem Sensor zum Messen einer Blutzuckermenge, einem Sensor zum Messen einer Blutharnstoffmenge oder einem Sensor zum Messen einer Blutkreatininmenge innerhalb des Filtrationssystems (210) oder des Reabsorptionssystems (220) umfasst.

11. System nach einem der obigen Ansprüche, das ferner Folgendes umfasst: einen Sensor zum Anzeigen einer Verunreinigung oder eines Bruchs des Nicht-Silizium-Keramikfilters im Filtrationssystem oder einen Sensor zum Anzeigen einer Verunreinigung oder eines Bruchs des Nicht-Silizium-Keramikfilters im Reabsorptionssystem.

12. System nach einem der obigen Ansprüche, das ferner Folgendes umfasst: eine Steuereinheit (160) mit einer elektronischen Anzeige und einer Computersteuerung in elektronischer Verbindung mit dem Einlassventil des Filtrationssystems, dem ersten Auslassventil des Reabsorptionssystems, dem zweiten Auslassventil des Reabsorptionssystems und der Filtratpumpe (250).

13. System nach Anspruch 12, wobei die Computersteuerung in elektronischer Verbindung mit einem oder mehreren der Sensoren der Ansprüche 9-11 ist.

14. System nach einem der obigen Ansprüche, das ferner eine Blutpumpe mit einem Einlass in Verbindung mit dem Arterientransplantat und einem Auslass in Verbindung mit dem Einlass an der Eingabeseite des Filtrationssystems umfasst.

15. System nach einem der obigen Ansprüche, das ferner wenigstens einen Batteriepack umfasst, der mit der Steuereinheit (160) verbunden ist.

## Revendications

1. Système de thérapie de remplacement rénal, comprenant :
un système de filtration (210) comportant un côté alimentation (211), un côté filtrat (212) et un filtre en céramique sans silicium (230) séparant le côté alimentation et le côté filtrat ;
un système de réabsorption (220) comportant un côté alimentation (222), un côté filtrat (221) et un filtre en céramique sans silicium (240) séparant le côté alimentation et le côté filtrat ;
un circuit de circulation sanguine sans valves en communication avec une greffe artérielle, une entrée vers le côté alimentation du système de filtration, une sortie vers le côté alimentation du système de filtration, une entrée vers le côté filtrat du système de réabsorption, une sortie vers le côté filtrat du système de réabsorption et une greffe veineuse ;
un tube introduit par voie percutanée (150) comprenant un canal d'alimentation en fluide, un canal de retour de fluide, un câble d'alimentation et un câble de communication de données ;
une valve d'entrée du système de filtration en communication avec le canal d'alimentation en fluide et une entrée vers le côté filtrat du système de filtration ;
une première valve de sortie du système de réabsorption en communication avec le côté alimentation du système de réabsorption et le canal de retour de fluide ;
une deuxième valve de sortie du système de réabsorption en communication avec le côté alimentation du système de réabsorption et une greffe en communication avec une vessie (103) ou une poche de collecte d'urine ;
une pompe à filtrat (250) comportant une entrée en communication avec le côté filtrat du système de filtration et une sortie en communication avec le côté alimentation du système de réabsorption ; et
un boîtier implantable contenant le système de filtration (210), le système de réabsorption (220) et la pompe à filtrat (250) .

2. Système selon la revendication 1, dans lequel le boîtier implantable est entièrement étanché à l'exception de quatre ouvertures.

3. Système selon la revendication 2, dans lequel les quatre ouvertures sont positionnées pour permettre une communication vers une partie intérieure du boîtier implantable pour le tube introduit par voie percutanée, l'entrée du circuit de circulation sanguine vers le côté alimentation du système de filtration (211), la sortie du circuit de circulation sanguine vers le côté filtrat du système de réabsorption (221), et une ouverture en communication avec la deuxième valve de sortie du système de réabsorption.

4. Système selon la revendication 1, dans lequel le filtre en céramique sans silicium du système de filtration (230) est sélectionné pour filtrer les composants sanguins de moins de 66 500 daltons en les faisant passer dans le côté filtrat du système de filtration (212).

5. Système selon la revendication 1, dans lequel le filtre en céramique sans silicium du système de réabsorption (240) est dimensionné pour laisser passer les composants présents dans le côté alimentation en filtrat (211) ayant une masse moléculaire inférieure à 500 daltons dans le côté filtrat du système de réabsorption (221) et dans le circuit de circulation sanguine sans valves.

6. Système selon la revendication 1, dans lequel le filtre en céramique sans silicium du système de filtration (230) est sélectionné pour retenir la quasi-totalité de l'albumine dans le sang qui s'écoule dans le circuit de circulation sanguine sans valves, s'écoulant à partir de l'entrée vers le côté alimentation du système de filtration, par la sortie vers le côté alimentation du système de filtration, tout en laissant passer vers le côté filtrat du système de filtration (212) les fluides à masse moléculaire ayant une masse moléculaire inférieure à 66 500 daltons.

7. Système selon l'une quelconque des revendications précédentes, dans lequel le filtre en céramique sans silicium du système de filtration (230) et/ou le filtre en céramique sans silicium du système de réabsorption (240) contiennent/contient un ou plusieurs de l'oxyde d'aluminium, de l'oxyde de zinc, de l'oxyde de titane, et du pyrocarbone.

8. Système selon l'une quelconque des revendications précédentes, dans lequel le filtre du système de filtration (230) contient un ou plusieurs de matériaux et structures ayant des valeurs de ténacité à la rupture K_{IC} supérieures à 1,00 (MPa•m^{1/2}).

9. Système selon la revendication 1, comprenant en outre un ou plusieurs d'un capteur pour mesurer une pression dans le système de filtration (210) ; un capteur pour mesurer un écoulement dans le système de filtration ; un capteur pour mesurer une pression dans le système de réabsorption (220) ; ou un capteur pour mesurer un écoulement dans le système de réabsorption.

10. Système selon la revendication 1, comprenant en outre un ou plusieurs d'un capteur pour mesurer une quantité d'une protéine sanguine, un capteur pour mesurer une quantité d'un glucose sanguin, un capteur pour mesurer une quantité d'une urée sanguine, ou un capteur pour mesurer une quantité d'une créatinine sanguine dans le système de filtration (210) ou le système de réabsorption (220) .

11. Système selon l'une quelconque des revendications précédentes, comprenant en outre un capteur pour indiquer un encrassement ou une rupture du filtre en céramique sans silicium dans le système de filtration ou un capteur pour indiquer un encrassement ou une rupture du filtre en céramique sans silicium dans le système de réabsorption.

12. Système selon l'une quelconque des revendications précédentes, comprenant en outre : une unité de commande (160) comportant un affichage électronique et un contrôleur informatique en communication électronique avec la valve d'entrée du système de filtration, la première valve de sortie du système de réabsorption, la deuxième valve de sortie du système de réabsorption et la pompe à filtrat (250) .

13. Système selon la revendication 12, dans lequel le contrôleur informatique est en communication électronique avec un ou plusieurs des capteurs selon les revendications 9 à 11.

14. Système selon l'une quelconque des revendications précédentes, comprenant en outre une pompe sanguine comportant une entrée en communication avec la greffe artérielle et une sortie en communication avec l'entrée vers le côté alimentation du système de filtration.

15. Système selon l'une quelconque des revendications précédentes, comprenant en outre au moins un bloc-batterie connecté à l'unité de commande (160).
